Europäisches Patentamt

European Patent Office

Office européen des brevets

(11) Publication number: **0 204 858**
**B1**

# EUROPEAN PATENT SPECIFICATION

(45) Date of publication of patent specification: **20.09.89**

(51) Int. Cl.⁴: **G 09 B 3/06,** G 09 B 19/06

(21) Application number: **85107267.8**

(22) Date of filing: **12.06.85**

(54) **Apparatus and method for cloze-elide testing.**

(43) Date of publication of application:
**17.12.86 Bulletin 86/51**

(45) Publication of the grant of the patent:
**20.09.89 Bulletin 89/38**

(84) Designated Contracting States:
**AT BE CH DE FR GB IT LI LU NL SE**

(56) References cited:
**US-A-3 900 961**
**US-A-4 300 123**
**US-A-4 419 080**
**US-A-4 481 412**

(73) Proprietor: **Educational Testing Service**
**Princeton, NJ 08540 (US)**

(72) Inventor: **Manning, Winton H.**
**12 Morven Place**
**Princeton, NJ 08540 (US)**

(74) Representative: **Ebbinghaus, Dieter et al**
**v. FÜNER, EBBINGHAUS, FINCK Patentanwälte**
**European Patent Attorneys Mariahilfplatz 2 & 3**
**D-8000 München 90 (DE)**

Courier Press, Leamington Spa, England.

## Description

This invention relates to a method of cloze testing as described in the pre-characterizing portion of Claim 1. Such method is known from the document: Mullen, "An Alternative to the Cloze Test", Tesol, presented to the 13th Annual Convention of Teachers of English Speakers of Other Languages, Boston, Mass., February 27 to March 4, 1979, p. 187 (1979).

The "Cloze" procedure as a form of testing was introduced in a publication by Wilson Taylor in 1953. Originally developed as an improved method of assessing text readability, cloze testing involves the presentation of a prose passage from which words have been deleted, thereby leaving a series of gaps or spaces in the text. The examinee is asked to fill in the blank corresponding to the deleted word by supplying the missing word or a reasonable substitute for it. Different algorithms can be devised for deletion of every nth word; deletion corresponding to particular parts of speech; or random deletion of words. A large number of public studies of the cloze approach to testing have helped establish its validity as a measure of reading comprehension that is in several ways superior to conventional tests, e.g. those which rely on asking multiple choice questions. However, the procedure has a fundamental problem in that it requires scoring by "exact word" or "acceptable word" rules.

The traditional open-ended or "completion cloze" test methods have obvious short comings in large scale testing programs, in that they are not readily amenable to machine scoring such as by optical scanners. In order to render cloze testing amenable to machine scoring, "multiple choice cloze" approaches have been developed. In this application, examinees are presented with four or five choices corresponding to each deleted word, and are asked to choose the best alternative word. However, this procedure has been criticized because the task is fundamentally one of recognizing the correct word, as opposed to producing it.

The subject matter of this invention encompasses specific developments of two formats that differ from the traditional cloze testing procedures, and which are adapted particularly for automatic scoring and/or presentation through a terminal screen. These formats are the "cloze-edit" procedure, and the "maze" procedure. In both of these procedures the test consists of a reading passage into which has been inserted, preferably randomly, words which are extraneous to the text. The task of the examinee is to elide, or strike out, the words that have been inserted into the running text. As a generic term for this general approach, I use the term Cloze-Elide testing. Although other alternative forms are possible, the invention is disclosed here only in terms of these two procedures, and particularly with respect to development of the cloze-edit procedure for automatic test scoring. The cloze edit technique in this general form has been discussed in the published literature. Reference is made to the publication of Davies in Testing Language Proficiency, edited by Jones and Spolsky, Center for Applied Linguistics, pp. 119—130 (1975); Bowen, The Identification of Irrelevant Lexical Distraction: An Editing Task, TESL Reporter, Vol. 12, No. 1, pp. 1—3, 14—16 (1978); and Mullen, An Alternative To The Cloze Test, Tesol presented to the 13th Annual Convention of Teachers of English Speakers of Other Languages, Boston, Mass., February 27 to March 4, 1979, p. 187 (1979).

In one embodiment of my development of the cloze edit test, specially prepared passages with distractor words inserted therein are printed on optically scannable answer sheets, and the examinee is required to eliminate the incorrect distractor words from the text by drawing a line through the words with a pencil or other marking device which generates a mark with a reflectivity which is distinguishable from that of the printed text. Each text word, and particularly the distractor words which are to be deleted, are printed at precisely predetermined locations on the page, and programmably controlled optical scanner apparatus is utilized to determine which distractor words had been properly elided and which other text words have been incorrectly elided. Alternately, as set forth in greater detail under the Description of the Preferred Embodiments, the test passage may be presented electronically, such as on a video screen, with the examinee being provided means for electronically deleting the distractor words.

The maze technique has been disclosed in a series of studies by Guthrie (1973); Guthrie, Siefert, Bernum and Caplin (1974); Fitzgerald & Fitzgerald (1978); and Pikulski (1977). In this general format, target words are selected from the given text, and two or more incorrect (foil) words are paired with the target word to form a triplet. The student then must choose from the presented options the word that is most appropriate. Generally, the triplets are chosen so that one of the "foil" words is in the same word class whereas a third word is in a different class. In this way, it is possible to distinguish between lexical and syntactical errors in reading the passage. While this format has the scoring advantage of a traditional multiple choice test, it has not heretofore been developed so as to be susceptible to automatic machine scoring. Furthermore, US—A—3 900 961 discloses a method for multiple choice tests involving a limited number of questions. The test may be formatted in the horizontal or vertical configurations. The marked answer sheets are fed by hand, one at a time, onto an optical scanner and then carried past a sensing station of five pencil-mark sensor and one control-mark sensor. The scoring method provides means for counting the correct answer and automatically printing their number at the bottom of the answer sheet. Incorrect answers are indicated but not counted, and omitted answers appear to be ignored. Although it is conceivable that one word could be printed in each of the available response positions on the test sheet, the result would be a list of approximately 50 words not connected into sentences by association or punctuation. Furthermore, a cloze-elide test of 50 words would be much too short; normally about 200 words or more are required. In addition, this method permits only one count of correct responses which has to be manually transcribed from each sheet for reporting. No counts of incorrect responses are supplied; incorrect responses would have to be counted visually from each·sheet and manually transcribed. With only one count of correct responses, there would be no way to distinguish a correctly overmarked distractor word from a correctly unmarked non-distractor word. Likewise, there would be no way to distinguish an incorrectly unmarked distractor word from an incorrectly overmarked non-distractor word. Accordingly, the method of test scoring as described above is entirely unsuitable for automatic processing of cloze-elide test sheets.

It is an object of this invention to provide a method for automatic machine scoring of Cloze-Elide tests.

It is another object of this invention to provide a method for automatic scoring of a Cloze-Elide test administered on a sheet.

It is another object of this invention to provide a method for automatic scoring of a Cloze-Elide test displayed on a video terminal.

It is another object of this invention to provide a method of automatic presentation of a Cloze-Elide test on a video terminal with electronic means for test taking by manipulation of the terminal display.

It is another object of this invention to provide a method for automatic scoring of an electronically presented Cloze-Elide test.

It is another object of this invention to provide a method of generating a Cloze-Elide test for automatic presentation upon demand.

It is a still further object of this invention to provide a method of selecting answers to a Cloze-Elide test presented on a video terminal, and of automatic scoring of the selected answers.

In accordance with the above objects, the invention resides in a method of cloze testing of the kind referred to in Claim 1.

Particular embodiments of the invention are let out in the dependent Claims 2 to 10.

According to the invention, a Cloze-Elide automated testing method is provided, using a display surface in the form either of a printed sheet or electronic display such as a video terminal, the display surface containing a text of words, the text having distractor words inserted therein which are to be elided by the person taking the test, the inserted distractor words being positioned on the surface at predetermined surface positions, the method further using storage for electronically storing data representative of the predetermined distractor word positions. Following selection of the words to be elided by the test taker, such as crossing out the elided words on the printed sheet or removing them electronically from the video display, means are provided for determining the positions or identity of the word selected to be elided from the text and for generating data representative of the elided words or word positions. Means for comparing the predetermined distractor word positions and the determined elided positions are provided, along with automatic computing means for providing an indication of the score made by the test taker. The invention will now be described by way of example with reference to the accompanying drawings, in which:

Figure 1A illustrates an unmarked test portion of the test in the Cloze-Elide test format.

Figure 1B shows the same test portion as Figure 1A, with overmark indicia indicating words selected to be elided by the test taker.

Figure 1C shows an unmarked test portion of a Maze test embraced by this invention.

Figure 2 shows a block diagram of a system for a Cloze-Elide testing method according to the invention utilizing a test sheet and scanning means for determining the elided words, and computing apparatus for computing the test score.

Figure 3 is a block diagram indicating the basic elements of an electronic system for a Cloze-Elide testing method, utilizing a video terminal, a CPU such as a microprocessor, and automatic elide means for manipulation of the text display on the video terminal.

Figure 4A is a block diagram illustrating the steps taken in a method of this invention for presenting and scoring a Cloze-Elide test.

Figure 4B is a flow diagram of a method of this invention for generating, displaying and scoring a Cloze-Elide test.

Figure 4C is another embodiment of a method of this invention for generating, displaying and scoring a Cloze-Elide test.

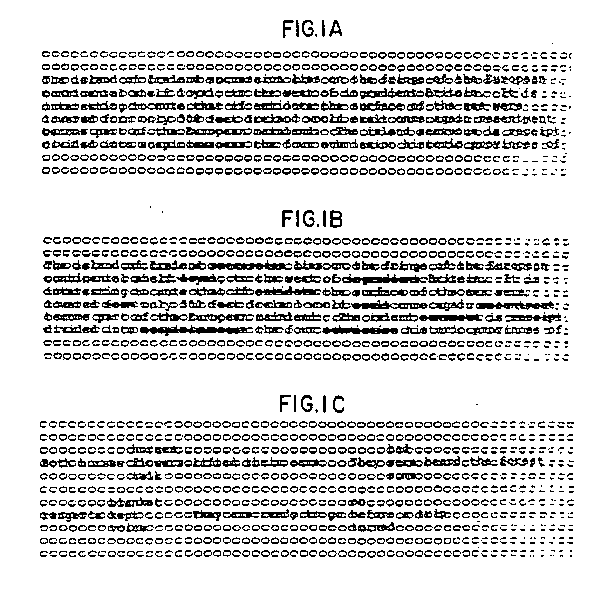

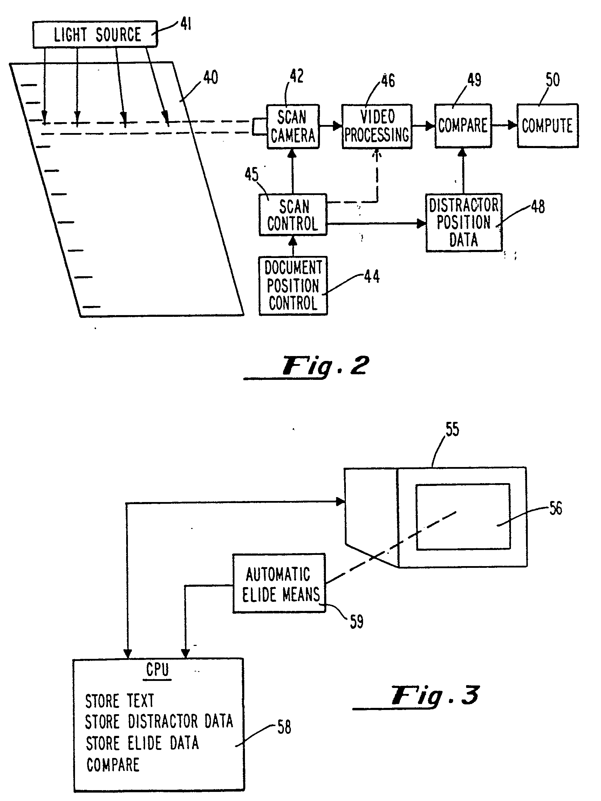

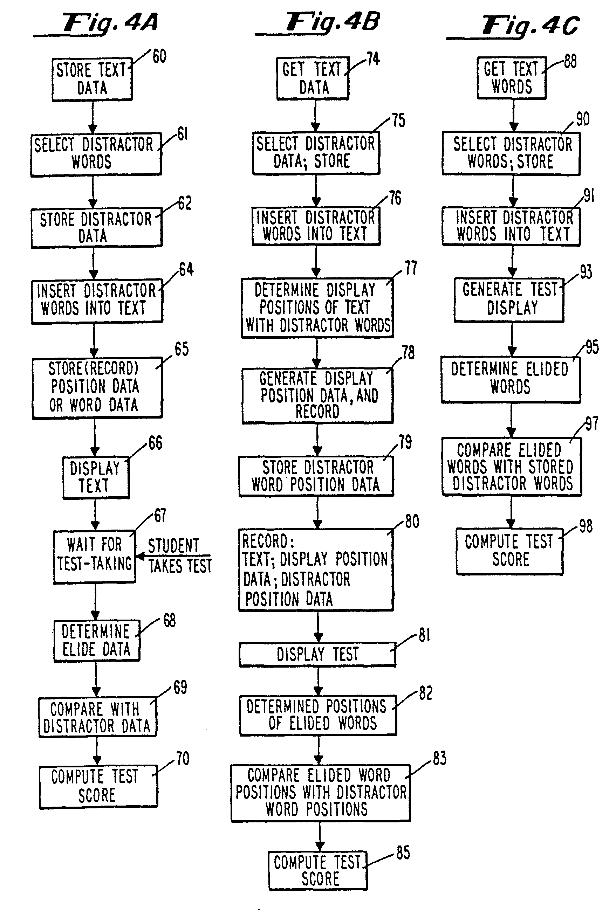

Referring now tv Figures 1A—1C, there are illustrated two forms of Cloze-Elide test presented in standard sheet form. Referring first to Figure 1A, there is illustrated a text 30 which is typed on a sheet having rows of marking areas 33, each marking area having a predetermined position with respect to the dimensions of the sheet. As seen, each word of the text covers one or more position areas, and thus is identified with one or more predetermined position areas. As set forth in the discussion above of Cloze-Elide testing, selected distractor words 31 are introduced into the text, these being the words that the test taker is asked to identify and elide. As illustrated in Figure 1B, the test taker has elided distractor words by providing an indicia, suitably in the form of a pencil marking of predetermined optical reflectivity, which is capable of being sensed by scan camera type of equipment. In practice, the marking areas 33, shown as approximately oval in form and sometimes referred to as "bubbles", are printed in an ink of a first optical reflectivity, to which the scanning equipment is substantially insensitive. For example, the mark areas may be printed in red ink. This contrasts with the pencil marking made by the test taker, with a type of lead which has a very reduced optical reflectivity compared to the rest of the test sheet, such that the mark is clearly sensed by the scanning equipment adopted for use with the system. Also illustrated in Figures 1A and 1B are position marks, or row marks in the form of bars shown in the lefthand margin. These marks are used, in a manner well known in the art, for providing synchronizing signals to scanning equipment, to aid the scanning equipment in finding and identifying rows which are being scanned.

Figure 1C shows a portion of a Maze test text, printed on the same form of test sheet with bubble marking areas, or positions. As with the Cloze-Edit test, a student eliminates the distractor or unwanted words, in this case by crossing out two of the three words presented. Of course, it is to be understood that other variations of the Cloze-Elide type test may be presented and utilized within the scope of this invention.

Referring now to Figure 2, there is shown a block diagram of a system used in connection with the method according to this invention for automatic scoring of a Cloze-Elide test which has been presented and taken on a sheet 40, such as shown in Figures 1A and 1B. The sheet 40 is transmitted past a light source 41 by document handling means not shown. Such document handling means are well known in the art. A scan camera 42 is utilized to scan each successive row, under the control of scan control 45. Scan control 45 is suitably in turn controlled by document position control 44, which also controls the document handling means, the signal from document position control 44 synchronizing the scan control 45. Also, as previoiusly indicated, row marks 34 may be utilized to further aid the scanning synchronization process. Applicant refers to U.S. Patent US—A—4,300,123, which shows a typical type of scanning system employing a light source and scan camera, for generating video data representative of marked test sheets. Reference is made to this patent for disclosure of a typical prior art scanning system which can be utilized in the practice of this invention. As is further shown in Figure 2, the video data from block 42 is processed at block 46 to provide video signals, suitably in digital form, representative of markings found in each successive row as it is scanned. The processing of 46 may also suitably be controlled by signals from scan control 45.

In practice of the method of this invention, the distractor position data, representing the distractor words 31 inserted into the text, is stored in memory 48. This data is inputted to a compare circuit, or program computer 49, for performing a comparison function. The distractor position data is compared with synchronized data from the video processing block 46. By this means, the apparatus or block 49 can determine whether the distractor words have been properly identified, and also determine whether other words not inserted as distractor words have been selected by the student. The results of the comparisons at block 49 are computed at block 50 to determine the test score. The computing steps of block 50 may be made by a separate digital circuit of standard design, but more preferably are carried out within the same programmed computing apparatus as the comparison steps of block 49. In the preferred embodiment, the computer, or CPU, may be a commercially available microprocessor based small computer. The stored distractor position data may be in any readily available form, such as read only memory (ROM), PROM, or any other medium. The actual architecture of the apparatus of Figure 2 is not essential to the invention, but it is essential to the embodiment of Figure 2 to have the stored distractor position data so as to obtain an accurate comparison with the video signals generated by the scan camera.

Referring to Figure 3, there is shown a simplified block diagram of an embodiment utilizing the video terminal 55 having a video display 56 of a known type. The video terminal 55 receives signals from a CPU 58, again in a known manner. The manner of controlling a video display from stored data in a CPU is well known in the art. At the start of the test, the CPU contains the stored text for display, as well as distractor data, either in the form of the distractor words or the form of the locations of the display distractor words, or both. The test taker uses an automatic elide means, illustrated at 59, to select and elide words which are identified as distractor words. Such automatic elide means may be any one of a number of commercially available means. Touch screen apparatus may be used, in which procedure the student or test taker merely touches the screen at the position of the word to be deleted, whereupon the stored program in CPU 58 causes the word to be deleted from the text. A light pen may be utilized, which technique is similar to the touch screen but incorporates the use of a light pen to delete the selected word or words. Alternately, a Koala pad may be used, with which a cursor is moved about on the video terminal screen by the student moving his or her finger across a specially designed pad which is an analog of the screen itself. When the cursor is properly located, the distractor words can be deleted or identified. Alternately, a "mouse" can be used, a device that is moved about on a desk top so as to control a cursor on the screen. The preferred embodiment employs a computer based technique such as illustrated in Figure 3 for response to the test, so as to make available a simple, direct and rapid resqonse mode which facilitates student motivation and enables the text processing task to be completed in a real time frame that is comaptible with the act of reading and editing a text.

Referring now to Figure 4A, there is shown a flow diagram of a method carried out by the system of either Figure 2 or Figure 3. As illustrated at 60, the text data is first stored in any convenient form. It may be previously stored on magnetic tape or a disk and transferred into a computer memory, or it may be available in any other convenient medium. For example, in the embodiment of Figure 2, it could be stored solely in a wordprocessor typewriter. At block 61, distractor words are selected in the fashion as discussed in the section titled Background of the Invention. Distractor data representing the selected distractor words is then stored or placed on a convenient medium, as shown at block 62. The next step is to insert the selected words into the text, as shown at 64. This may be done semiautomatically such as by an operator inserting the words into the text using a wordprocessor. Alternately, it may be done automatically by a software subroutine which operates under a control algorithm to insert the distractor words at different places in the text. Following this, position data is generated corresponding to the words of the text, and stored as indicated at 65. Thus, for a software controlled system, i.e., one where the text will be displayed on a video terminal, information is generated which is used to control display of the text such that each word occurs at a predetermined and known position, which positions are then recorded. In particular this step involves recording or storing position data corresponding to the distractor words which were inserted at step 64 into the text. Alternately, for a system of the embodiment of Figure 3, word data corresponding to the distractor words themselves may be stored, for later comprison with elided words, i.e. a word-to-word comparison as contrasted to a position comparison.

Continuing with Figure 4A, as illustrated at 66, the text is displayed, in any of the forms as has been discussed hereinabove. The method then entails waiting for the test taking, which is done by the student or person who actually takes the test. At this point, the manner of eliding is determined by the type of system being used, again as discussed above. After the test has been taken, at block 68 the system determines what data has been elided. This may be done either by determining the positions of elided data, or by determining the actual words that have been elided. In either event, the step embraces generating electronic signals representative of the elided data. This electronic elide data is then compared with the distractor data that had been stored at 62 and/or 65, as illustrated at block 69. This comparison may be a position-to-position comparison, or a word-to-word comparison. It is preferably done automatically in accordance with conventional software technique. Following this comparison, a test score is computed at block 70, in accordance with any desired algorithm. For a Cloze-Edit test, the algorithm preferably includes a compilation of the number of distractor words correctly elided, as well as a compilation of the number of other text words which should not have been elided but which were elided by the student. For example, the test score may be computed by simple difference determination of the number of correctly elided words minus the number of incorrectly elided words. Other variations may be adopted within the scope of the invention.

Referring now to Figure 4B, there is indicated a flow diagram of a method which incorporates initial generation of a test, wherein the scoring is done on a position comparison basis. As indicated at block 74, the first step is to get the text data. Next, at 75, the distractor data corresponding to selected distractor words is obtained and stored. Following this, at 76, the distractor words are inserted into the text, in a manner as discussed above. Thus, following step 76, for an electronic embodiment there has been obtained stored data which can be sequentially read to provide the text to be presented to the test taker, the text having inserted therein the selected distractor words. Next, at block 77, the display positions of the text with the distractor words are determined. In other words, there is a determination of where, for example, the text will be displayed on a video terminal, i.e., where it will be placed on a row-by-row and word-by-word basis. The method then proceeds to generate display position data corresponding to the determined display positions and to record or store such data as indicated at block 78. Thus, electronic signals are generated which can be used to control display of the text such that all words, and particularly the distractor words, appear at predetermined known locations on the display. Thus, no matter what the display device, the method embraces presenting the display in a predetermined spatial manner, such that the system knows exactly where each word is located. Specifically, as indicated at block 79, the distractor word position data is stored for later use in scoring the test after the student has taken the test. As indicated at block 80, the text; the display position data; and the distractor position data are stored or recorded at block 80. This may suitably be done on magnetic tape, on a disk, or any type of cartridge which can conveniently be utilized for causing display of the test text, either on a video or like terminal, or on a printed sheet.

The next step in the method of Figure 4B is to actually display the test, under control of the data which has been recorded at block 80. Thus, a disk or cartridge, or PROM may be inserted into apparatus such as shown in Figure 3, to cause the text to appear on the video terminal. Alternately, the stored data, such as on magnetic tape, can be used to cause printout of a test sheet. Then, at block 82, after the student or test taker has taken the test, the positions of the elided words are automatically determined. These elided word positions are compared with the distractor word positions that have been stored at block 79, and the test score is computed in any desired manner, as described above in the context of the method of Figure 4A.

Referring now to Figure 4C, there is shown another block diagram of a method which incorporates generation of a Cloze-Elide test, and wherein the scoring is done by comparing distractor words with selected elided words, whether or not positions are compared. At block 88, the text words are obtained, and at block 90 the selected distractor words are obtained and stored. At block 91, the distractor words are inserted into the text in the manner as described above, and at block 93 the test is displayed, i.e. the full text with the inserted distractor words is displayed. Following this the student takes the test, and at block 95 the elided words are determined. For example, referring to Figure 1B, data reprasentative of the actual words that were crossed out is generated and held in storage. Then, at block 97, the elided word data is compared with the stored distractor words, and at block 98 the test score is computed.

The above descriptions are applicable to either type of Cloze-Elide test as discussed herein, i.e. either Cloze-Edit or Maze. Also, as used herein, the term "word" includes both the idea of words as normally considered in the context of written language, and also includes alpha-numeric words. For example, the system and method of this invention may embrace mathematical texts, or texts which include other than conventional language texts. As set forth in the background section, a preferred embodiment incorporates language testing, and in particular second language testing, i.e, testing of a student in a foreign language.

## Claims

1. A method of cloze testing which includes presenting a text (30) of words on a test display surface, said text having non-distractor words and inserted distractor words (31) therein which are to be elided, characterized in that said method includes:

electronically reading and storing the positions of said non-distractor words and said distractor words (31) with respect to said test display surface (56),

instructing a test taker to elide distractor words (31) from said displayed text,

electronically determining the positions of the non-distractor and distractor words (31) elided by the test taker and generating data reprsentative of said elided positions, and

electronically comparing the determined elided positions with the stored distractor positions and automatically generating a score indicative of the accuracy of selection of said distractor words (31).

2. The method of Claim 1 further characterized in that said distractor words (31) are distributed randomly within the text (30).

3. The method of Claim 1 further characterized in that said method includes displaying said text on a video terminal (55).

4. The method of Claim 2 further characterized in that said displaying step includes providing a numerical score.

5. The method of Claim 4 further characterized in that said determining step includes automatically determining the positions of the elided non-distractor and distractor words (31), automatically comparing the elided word positions with the stored distractor positions, and automatically computing a test score indicative of the test taker's accuracy in properly eliding the distractor words (31).

6. The method of Claim 5 further characterized in that said computing step includes determining which distractor words (31) have been properly elided and which non-distractor words have been improperly elided.

7. The method of Claim 6 further characterized in that said storing step includes storing said text in an electronic memory (58).

8. The method of Claim 1 further characterized in that said words comprise numerals.

9. The method of Claim 5 further characterized in that said computing step includes subtracting a number of incorrectly elided words from a number of correctly elided words.

10. The method of Claim 5 further characterized in that said computing step includes compiling a number of elided non-distractor words that should not have been elided by the test taker.

## Patentansprüche

1. Verfahren zur Durchführung von Cloze-Prüfungen, das die Darbietung eines Textes (30) mit Wörtern auf einer Prüfungsanzeigefläche einschließt, wobei der Text nichtverwirrende Wörter und darin eingefügte verwirrende Wörter (31), die elidiert werden sollen, aufweist, dadurch gekennzeichnet,

daß das Verfahren einschließt:

das elektronische Lesen und Speichern der Positionen der nicht-verwirrenden Wörter und der verwirrenden Wörter (31) bezüglich der Prüfungsanzeigefläche (56),

das Anweisen eines Prüflings, die verwirrenden Wörter (31) aus dem angezeigten Text zu elidieren,

das elektronische Bestimmen der Positionen der nicht-verwirrenden Wörter und der vom Prüfling elidierten, verwirrenden Wörter (31) und das Erzeugen von Daten, die für die elidierten Positionen repräsentativ sind, und das elektronische Vergleichen der bestimmten, elidierten Position mit den gespeicherten Verwirr-Positionen und das automatische Erzeugen einer Bewertung, die die Genauigkeit der Auswahl der verwirrenden Wörter (31) anzeigt.

2. Verfahren nach Anspruch 1, ferner dadurch gekennzeichnet, daß die verwirrenden Wörter (31) willkürlich im Text (30) verteilt sind.

3. Verfahren nach Anspruch 1, ferner dadurch gekennzeichnet, daß das Verfahren das Anzeigen des Textes auf einem Videoterminal (55) einschließt.

4. Verfahren nach Anspruch 2, ferner dadurch gekennzeichnet, daß der Anzeigeschritt das Vorsehen einer numerischen Bewertung einschließt.

5. Verfahren nach Anspruch 4, ferner dadurch gekennzeichnet, daß der Bestimmungsschritt das automatische Bestimmen der Positionen der elidierten, nicht-verwirrenden Wörter und der verwirrenden Wörter (31), das automatische Vergleichen der Positionen der elidierten Wörter mit den gespeicherten Positionen der verwirrenden Wörter und das automatische Berechnen eines Prüfungsergebnisses einschließt, das auf die Genauigkeit des Prüflings beim richtigen Elidieren der verwirrenden Wörter (31) hinweist.

6. Verfahren nach Anspruch 5, ferner dadurch gekennzeichnet, daß der Berechnungsschritt die Bestimmung einschließt, welche verwirrenden Wörter (31) richtig elidiert wurden und welche nicht-verwirrenden Wörter falsch elidiert wurden.

7. Verfahren nach Anspruch 6, ferner dadurch gekennzeichnet, daß der Bewertungsschritt das Speichern des Textes in einem elektronischen Speicher (58) einschließt.

8. Verfahren nach Anspruch 1, ferner dadurch gekennzeichnet, daß die Wörter Zahlen aufweisen.

9. Verfahren nach Anspruch 5, ferner dadurch gekennzeichnet, daß der Berechnungsschritt das Substrahieren einer Anzahl von falsch elidierten Wörtern von einer Anzahl von richtig elidierten Wörtern einschließt.

10. Verfahren nach Anspruch 5, ferner dadurch gekennzeichnet, daß der Berechnungsschritt das Kompilieren einer Anzahl von elidierten, nicht-verwirrenden Wörtern einschließt, die der Prüfling nicht hätte elidieren sollen.

**Revendications**

1. Procédé pour faire un examen du type cloze, comportant le fait de présenter un texte (30) de mots sur une surface de visualisation de l'examen, ledit texte contenant des mots non détracteurs et des mots (30) détracteurs, qu'il faut supprimer, caractérisé en ce que ledit procédé consiste à:

lire et mémoriser par voie électronique les positions desdits mots non détracteurs et desdits mots (31) détracteurs, par rapport à ladite surface (56) de visualisation de l'examen,

demander au candidat qui passe l'examen de supprimer les mots (31) détracteurs dans ledit texte visualisé,

déterminer par voie électronique les positions des mots non détracteurs et des mots (31) détracteurs et supprimés par le candidat qui passe l'examen, et générer des données représentatives desdites positions supprimées, et

comparer par voie électronique, les positions supprimées déterminées avec les positions détractrices et mémorisées et générer automatiquement une cotation indicative de la précision du choix desdits mots (31) détracteurs.

2. Procédé selon la revendication 1, caractérisé en outre en ce que lesdits mots (31) détracteurs sont distribués de façon aléatoire à l'intérieur du texte (30).

3. Procédé selon la revendication 1, caractérisé en outre en ce que ledit procédé inclut le fait de visualiser ledit texte sur un terminal vidéo (55).

4. Procédé selon la revendication 2, caractérisé en outre en ce que ledit pas de visualisation inclut le fait de fournir une cotation numérique.

5. Procédé selon la revendication 4, caractérisé en outre en ce que ledit pas de détermination inclut de déterminer automatiquement les positions des mots non détracteurs qui ont été supprimés et des mots (31) détracteurs qui ont été supprimés, de comparer automatiquement les positions des mots supprimés avec les positions, mémorisées, des mots détracteurs et de calculer automatiquement une cotation d'examen indicative de la précision avec laquelle le candidat qui a passé l'examen a correctement supprimé les mots (31) détracteurs.

6. Procédé selon la revendication 5, caractérisé en outre en ce que ledit pas de traitement inclut le fait de déterminer quels sont les mots (31) détracteurs qui ont été correctement supprimés et quels sont les mots non détracteurs qui ont été incorrectement supprimés.

7. Procédé selon la revendication 6, caractérisé en outre en ce que ledit pas de mémorisation inclut le fait de mémoriser ledit texte dans une mémoire électronique (58).

8. Procédé selon la revendication 1, caractérisé en outre en ce que lesdits mots comportent aussi les chiffres.

9. Procédé selon la revendication 5, caractérisé en outre en ce que ledit pas de traitement inclut le fait de soustraire un nombre de mots incorrectement supprimés d'un nombre de mots correctement supprimés.

10. Procédé selon la revendication 5, caractérisé en outre en ce que ledit pas de traitement inclut le fait de compiler un nombre de mots non détracteurs et qui ont été supprimés et qui n'auraient pas dû être supprimés par le candidat qui a passé l'examen.

## FIG.1A

## FIG.1B

## FIG.1C

_Fig. 2_

_Fig. 3_

EP 0 204 858 B1

**_Fig. 4A_**

STORE TEXT DATA — 60

SELECT DISTRACTOR WORDS — 61

STORE DISTRACTOR DATA — 62

INSERT DISTRACTOR WORDS INTO TEXT — 64

STORE(RECORD) POSITION DATA OR WORD DATA — 65

DISPLAY TEXT — 66

WAIT FOR TEST-TAKING — 67 ← STUDENT TAKES TEST

DETERMINE ELIDE DATA — 68

COMPARE WITH DISTRACTOR DATA — 69

COMPUTE TEST SCORE — 70

**_Fig. 4B_**

GET TEXT DATA — 74

SELECT DISTRACTOR DATA; STORE — 75

INSERT DISTRACTOR WORDS INTO TEXT — 76

DETERMINE DISPLAY POSITIONS OF TEXT WITH DISTRACTOR WORDS — 77

GENERATE DISPLAY POSITION DATA, AND RECORD — 78

STORE DISTRACTOR WORD POSITION DATA — 79

RECORD: TEXT; DISPLAY POSITION DATA; DISTRACTOR POSITION DATA — 80

DISPLAY TEST — 81

DETERMINED POSITIONS OF ELIDED WORDS — 82

COMPARE ELIDED WORD POSITIONS WITH DISTRACTOR WORD POSITIONS — 83

COMPUTE TEST SCORE — 85

**_Fig. 4C_**

GET TEXT WORDS — 88

SELECT DISTRACTOR WORDS; STORE — 90

INSERT DISTRACTOR WORDS INTO TEXT — 91

GENERATE TEST DISPLAY — 93

DETERMINE ELIDED WORDS — 95

COMPARE ELIDED WORDS WITH STORED DISTRACTOR WORDS — 97

COMPUTE TEST SCORE — 98

3